(19) **Europäisches Patentamt**

**European Patent Office**

**Office européen des brevets**

(11) **EP 1 008 340 A2**

(12) # DEMANDE DE BREVET EUROPEEN

(43) Date de publication:
**14.06.2000 Bulletin 2000/24**

(51) Int Cl.⁷: **A61K 7/48**

(21) Numéro de dépôt: **99401957.8**

(22) Date de dépôt: **30.07.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **18.09.1998 FR 9811689**

(71) Demandeur: **L'OREAL**
**75008 Paris (FR)**

(72) Inventeurs:
• **Touzan, Philippe**
**75012 Paris (FR)**

• **Delambre, Patricia**
**94480 Ablon (FR)**
• **Bazin, Roland**
**91570 Bièvres (FR)**
• **Augot, Sandra**
**93140 Bondy (FR)**

(74) Mandataire: **Rasson, Catherine**
**L'OREAL-DPI**
**6 rue Bertrand Sincholle**
**92585 Clichy Cedex (FR)**

(54) **Utilisation à pH acide de silicates mixtes en tant qu'agents tenseurs dans des compositions cosmétiques anti-rides**

(57) L'invention concerne l'utilisation de silicates mixtes capables de former, après application à une concentration de 3 % dans l'eau à pH acide puis séchage, un film perméable à la vapeur d'eau produisant une rétraction du *stratum corneum* isolé supérieure à 1,5 % à une température de 30 °C et pour une humidité relative de 40 %, en tant qu'agents tenseurs pour tendre la peau et la lisser en vue d'atténuer immédiatement les rides et/ou ridules.

Elle concerne également les compositions cosmétiques contenant ces silicates mixtes à pH acide.

EP 1 008 340 A2

## Description

La présente invention concerne l'utilisation de silicates mixtes dans des compositions cosmétiques à pH acide destinées à diminuer et/ou à effacer les rides et/ou les ridules de la peau par un effet tenseur, les compositions cosmétiques anti-rides comprenant ces silicates mixtes à effet tenseur ainsi qu'un procédé de traitement cosmétique utilisant ces compositions.

Le processus de vieillissement de la peau s'accompagne d'une modification progressive de la structure et des fonctions cutanées. Les principaux signes cliniques du vieillissement cutané sont l'apparition de rides et de ridules qui augmentent avec l'âge.

Il est connu de remédier à ces signes du vieillissement en utilisant des compositions cosmétiques ou dermatologiques contenant des principes actifs tels que les $\alpha$-hydroxy-acides, les $\beta$-hydroxy-acides et les rétinoïdes. Ces molécules agissent sur les rides en éliminant les cellules mortes et en accélérant le processus de renouvellement cellulaire. Toutefois, l'effet visible de ces compositions ne se produit qu'au bout d'un certain temps d'application, pouvant aller de quelques jours à plusieurs semaines.

Une approche pour résoudre ce problème a consisté à utiliser des agents dits "tenseurs" qui, par un effet de tension de la couche superficielle de la peau, sont capables de lisser la peau en diminuant le nombre et la profondeur des rides et ridules et de faire disparaître les marques de fatigue, et ceci de façon instantanée.

Les composés tenseurs connus sont généralement des composés organiques macromoléculaires tels que des protéines, des dérivés de chitine ou des latex.

La demanderesse a découvert de manière surprenante que certains composés minéraux, à savoir des silicates mixtes, en dispersion dans un milieu aqueux acide possédaient un excellent pouvoir lissant instantané de la couche superficielle de la peau et pouvaient donc être utilisés en tant qu'agents tenseurs dans des compositions cosmétiques anti-rides au même titre que les polymères tenseurs de l'art antérieur.

L'utilisation de silicates mixtes dans des compositions cosmétiques est décrite dans l'art antérieur.

Ces silicates, connus pour leurs propriétés thixotropes, peuvent être utilisés en tant qu'agents épaississants, filmogènes, émulsifiants, adsorbants et/ou absorbants.

Ainsi, le brevet WO-A-97/31619 décrit des compositions cosmétiques nettoyantes et astringentes renfermant, entre autres, de la laponite, un silicate de sodium, de magnésium et de lithium, utilisée pour ses propriétés d'absorption d'huile. Cette même laponite peut être utilisée en tant qu'agent épaississant et/ou gélifiant dans les compositions cosmétiques décrites dans la demande de brevet EP-A-412 449.

Aucun document de l'art antérieur ne décrit l'utilisation de silicates mixtes, et en particulier de laponite, pour leur pouvoir lissant de la peau.

La présente invention a donc pour objet l'utilisation, à pH acide, de silicates mixtes en tant qu'agents tenseurs pour tendre la peau et la lisser en vue d'atténuer immédiatement les rides et/ou ridules.

Elle a également pour objet l'utilisation, à pH acide, de silicates mixtes à effet tenseur pour la préparation d'une composition cosmétique destinée à diminuer et/ou effacer les rides et/ou ridules de la peau.

La présente invention a en outre pour objet des compositions cosmétiques anti-rides comprenant, dans un milieu acide, ces silicates mixtes à effet tenseur.

Un autre objet de l'invention est un procédé de traitement non thérapeutique de la peau avec les compositions cosmétiques anti-rides ci-dessus.

D'autres objets apparaîtront à la lecture de la description et des exemples qui suivront.

L'effet cosmétique des compositions de la présente invention repose sur leur pouvoir "tenseur" de la peau.

Ce pouvoir "tenseur" est défini selon la présente invention comme la capacité d'un film homogène formé sur le *stratum corneum* isolé, à provoquer une rétraction d'au moins 1,5 % de celui-ci à une température de 30 °C et pour une humidité relative de 40 %. Selon la présente invention, ce film est formé par application et séchage d'une dispersion aqueuse contenant 3 % dudit silicate mixte.

Pour déterminer l'effet tenseur, on mesure la longueur de l'échantillon de *stratum corneum* avant traitement et celle obtenue après traitement, c'est-à-dire après application du silicate mixte. L'effet tenseur est caractérisé par la diminution de la longueur de l'échantillon après traitement, rapportée à la longueur initiale de l'échantillon avant traitement, selon l'équation :

$$\text{effet tenseur} = 100 \times \frac{l_1 - l_0}{l_0}\ \%$$

$l_1$ étant la longueur après traitement et $l_0$ la longueur avant traitement. La longueur de l'échantillon est mesurée avec l'appareil "Miniature Tensile tester MTT 610".

Le film tenseur formé sur l'épiderme doit être perméable à la vapeur d'eau pour ne pas entraver la transpiration cutanée.

La perméabilité du film est évaluée par mesure de la *Perte Insensible en Eau* (PIE) du *stratum corneum* dégraissé et traité par la composition. Lorsque le film est suffisamment perméable à la vapeur d'eau la PIE n'est pas modifiée. La mesure de la PIE s'effectue de manière classique à l'aide d'un évaporimètre (Servomed) qui permet une détermination quantitative de l'évaporation d'eau, c'est-à-dire du transport d'eau par diffusion sur un échantillon de *stratum corneum* obturant une capsule cylindrique contenant de l'eau, le tout étant placé dans une chambre à température et humidité relative contrôlées. Des capteurs permettent de mesurer la pression partielle de vapeur d'eau en deux points situés à des distances différentes de l'échantillon. On détermine ainsi le gradient de pression partielle de vapeur d'eau entre les deux points, et donc le taux d'évaporation conformément à la loi de Fick.

On entend par silicates mixtes dans la présente invention tous les silicates d'origine naturelle ou synthétique renfermant plusieurs types de cations choisis parmi les métaux alcalins (par exemple Na, Li, K) ou alcalino-terreux (par exemple Be, Mg, Ca) et les métaux de transition.

Pour garantir de bonnes propriétés cosmétiques, ces minéraux doivent se présenter sous une forme finement divisée. On recommande en particulier des poudres de silicates dont les particules ont une taille moyenne comprise entre 15 nm et 1 μm (entre 15 et 1000 nm), et de préférence entre 20 nm et 600 nm. On entend ici par taille moyenne la dimension moyenne du diamètre des particules.

On utilise de préférence dans la présente invention des phyllosilicates, à savoir des silicates ayant une structure dans laquelle les tétraèdres SiO4 sont organisés en feuillets entre lesquels se trouvent enfermés les cations métalliques.

Une famille de silicates particulièrement préférée pour l'utilisation dans les compositions cosmétiques de la présente invention est celle des laponites. Les laponites sont des silicates de magnésium, de lithium et de sodium ayant une structure en couches semblable à celle des montmorillonites. La laponite est la forme synthétique du minéral naturel appelé "hectorite". L'origine synthétique de cette famille de silicates présente un avantage considérable par rapport à la forme naturelle car elle permet une bonne maîtrise de la composition du produit.

La laponite utilisée dans la présente invention est commercialisée par exemple sous la dénomination Laponite XLS ou Laponite XLG par la société SOUTHERN CLAY PRODUCTS.

La demanderesse a constaté que le pouvoir tenseur des silicates mixtes augmentait fortement lorsque ceux-ci se trouvaient en dispersion dans un milieu aqueux acide (voir Exemple 1). Le pH des compositions cosmétiques anti-rides de la présente invention doit par conséquent être inférieur à 7. Pour des raisons de tolérance cutanée, on préfère un pH modérément acide voisin de celui de la peau, à savoir un pH compris entre 5 et 6 et en particulier un pH d'environ 5,5.

Le bon pouvoir tenseur des silicates mixtes utilisés en milieu acide permet de les utiliser en des proportions relativement faibles. La teneur en silicate mixte des compositions cosmétiques de la présente invention est de préférence comprise dans un intervalle allant de 0,5 à 10 % en poids, et plus préférentiellement de 0,5 à 5 % en poids rapporté à la composition cosmétique totale.

Les compositions anti-rides selon l'invention peuvent contenir en outre un ou plusieurs principes actifs cosmétiques choisis parmi les agents anti-radicaux libres, les agents anti-UV, les agents hydratants, les vitamines, les protéines, les céramides, les α-hydroxy-acides, les β-hydroxyacides, les rétinoïdes et les polymères tenseurs notamment organiques tels que les latex, les hydrolysats de protéines et les dérivés de chitine.

Elles peuvent contenir en outre des adjuvants cosmétiques usuels en cosmétique. Ces adjuvants sont par exemple les solvants, les agents régulateurs de pH, les anti-oxydants, les agents séquestrants, les agents conservateurs, les pigments et colorants, les charges, les émollients, les agents anti-mousse, les corps gras tels que huiles ou cires végétales ou animales, les silicones, les parfums, les agents tensioactifs, les plastifiants et les polymères épaississants ou gélifiants.

Bien entendu, l'homme de métier veillera à choisir ce ou ces éventuels composés complémentaires et leur quantité, de manière telle que les propriétés avantageuses attachées intrinsèquement à la composition cosmétique ou dermatologique conforme à l'invention ne soient pas, ou substantiellement pas, altérées par la ou les adjonctions envisagées.

Les compositions anti-rides peuvent se présenter sous n'importe quelle forme permettant la formation d'un film homogène ayant l'effet cosmétique tenseur souhaité. Il s'agit par exemple d'une solution aqueuse ou hydroalcoolique, d'une émulsion huile-dans-eau ou eau-dans-huile ou encore d'une émulsion multiple, d'un gel aqueux, d'une dispersion d'huile dans une phase aqueuse en présence de sphérules, ces sphérules pouvant être des nanoparticules polymériques telles que les nanocapsules ou nanosphères, ou mieux des vésicules lipidiques de type ionique et/ou non ionique.

Cette composition peut être plus ou moins fluide et avoir l'aspect d'une crème, d'une pommade, d'un lait, d'une lotion, d'un sérum ou d'une mousse. Elle peut éventuellement être appliquée sur la peau sous forme d'aérosol. Elle peut également se présenter sous forme d'une pâte plus ou moins dure, par exemple sous forme de stick. Elle peut être utilisée comme produit de soin et/ou comme produit de maquillage de la peau.

La composition cosmétique anti-rides se présente plus particulièrement sous forme d'un sérum ou d'une crème huile-dans-eau.

L'invention est illustrée plus en détail dans les exemples suivants. Les quantités sont en pourcentage en poids, sauf mention contraire.

**Exemple 1**

On prépare différentes dispersions aqueuses de laponite qui diffèrent principalement par leur pH et leur force ionique. Ces dispersions sont appliquées sur des *stratum corneum* isolés et on mesure la rétraction de cette couche superficielle de la peau de la manière décrite ci-dessus. Les résultats sont rassemblés dans le tableau suivant :

|  | A | B | C | D | E |
|---|---|---|---|---|---|
| Laponite | 8,4 % | 8,4 % | 9,1 % | 8,4 % | 8,4 % |
| Mexoryl SX | - | 2 % | - | 2 % | |
| triéthanola mine | - | - | - | 0,4 % | - |
| NaCl | - | - | - | - | 2,7 % |
| acide acétique | 0,3% | - | - | - | - |
| eau | 91,3 % | 89,6 % | 90,9 % | 89,2 % | 88,9 % |
| pH | 5,8 | 5,7 | 9,3 | 8,2 | 8,6 |
| effet tenseur | -30,40 % | -41 % | -15,32 % | -18 % | -7,19 % |

Le Mexoryl SX est un filtre solaire constitué par l'acide benzène 1,4-di(3-méthylidène-10-camphosulfonique) commercialisé par la société CHIMEX. Ce composé a également des propriétés acides et permet de ce fait l'ajustement du pH de la composition B à une valeur inférieure à 7.

On constate que les deux dispersions à pH acide conformes à l'invention (A et B) ont un pouvoir tenseur nettement plus important que les compositions à pH faiblement à moyennement basique (C, D et E). Le faible pouvoir tenseur de la composition E confirme que l'augmentation de l'effet tenseur est dû à la diminution du pH et non pas à l'augmentation de la force ionique.

**Exemple 2**

On prépare un sérum anti-rides à partir des ingrédients suivants :

| Laponite XLS | 3 % |
|---|---|
| Conservateur | 0,3 % |
| Mexoryl SX | 0,7 % |
| Eau déminéralisée | q. s. 100 % |

Le sérum ainsi obtenu a un pH de 5,7

**Exemple 3**

On prépare une crème anti-rides à partir des ingrédients suivants :

| Alcool cétylique | 7 % |
|---|---|
| Huile de vaseline | 9 % |
| Stéarate de diglycéryle | 2,5 % |
| Stéarate de PEG-50 | 2,5 % |
| Laponite XLS | 5 % |
| Mexoryl SX | 0,7 % |
| Eau déminéralisée | q. s. 100 % |

La crème ainsi obtenue a un pH de 5,5.

**Exemple 4**

On prépare une solution anti-rides à partir des ingrédients suivants :

| Laponite XLS | 3 % |
|---|---|
| Gomme de xanthane | 0,5 % |
| Glycérine | 1,5 % |
| Acide citrique | 0,32 % |
| Eau déminéralisée | q. s. 100 % |

La solution ainsi obtenue a un pH de 5,5.

**Exemple 5**

On prépare une crème anti-rides contour des yeux à partir des ingrédients suivants :

| Alcool cétylique | 2 % |
|---|---|
| Huile de vaseline | 8 % |
| Tristéarate de sorbitane | 0,9 % |
| Stéarate de PEG-40 | 1 % |
| Stéarate de glycérol | 1 % |
| Huile végétale | 4 % |
| Cyclométhicone | 5 % |
| Laponite XLS | 5 % |
| Acide citrique | 0,4 % |
| Eau déminéralisée | q. s. 100 % |

La crème anti-rides ainsi obtenue a un pH de 5,5.

**Revendications**

1. Utilisation à pH acide d'un silicate mixte capable de former, après application à une concentration de 3 % dans l'eau à pH acide puis séchage, un film perméable à la vapeur d'eau produisant une rétraction du stratum corneum isolé supérieure à 1,5 % à une température de 30 °C et pour une humidité relative de 40 %, en tant qu'agent tenseur pour tendre la peau et la lisser en vue d'atténuer immédiatement les rides et/ou ridules.

2. Utilisation d'un silicate mixte capable de former, après application à une concentration de 3 % dans l'eau à pH acide puis séchage, un film perméable à la vapeur d'eau produisant une rétraction du stratum corneum isolé supérieure à 1,5 % à une température de 30 °C et pour une humidité relative de 40 %, pour la préparation d'une composition cosmétique destinée à diminuer et/ou effacer les rides et/ou ridules de la peau.

3. Utilisation selon la revendication 1 ou 2, caractérisée par le fait que le pH du milieu aqueux d'application est compris entre 5 et 6.

4. Utilisation selon l'une quelconque des revendications 1 à 3, caractérisée par le fait que le silicate mixte se présente sous une forme finement divisée ayant une taille moyenne de particules comprise entre 15 nm et 1000 nm.

5. Utilisation selon l'une quelconque des revendications 1 à 4, caractérisée par le fait que le silicate mixte est un phyllosilicate.

6. Utilisation selon la revendication 4, caractérise par le fait que le silicate mixte appartient à la famille des hectorites.

7. Utilisation selon la revendication 6, caractérisée par le fait que le silicate mixte est une laponite.

8. Utilisation selon l'une quelconque des revendications précédentes, caractérisée par le fait que le silicate mixte est présent à raison de 0,5 à 10 % en poids, de préférence à raison de 0,5 à 5 % en poids par rapport au poids total de la composition.

9. Composition cosmétique anti-rides, caractérisée par le fait qu'elle comprend, dans un milieu aqueux acide physiologiquement acceptable, un silicate mixte capable de former un film perméable à la vapeur d'eau et produisant, après application à une concentration de 3 % dans l'eau puis séchage, une rétraction du stratum corneum isolé supérieure à 1,5 %, à une température de 30 °C et pour une humidité relative de 40 %.

10. Composition cosmétique anti-rides selon la revendication 9, caractérisée par le fait que le pH du milieu aqueux acide physiologiquement acceptable est compris entre 5 et 6.

11. Composition cosmétique anti-rides selon la revendication 9 ou 10, caractérisée par le fait que le silicate se présente sous une forme finement divisée ayant une taille moyenne de particules comprise entre 15 nm et 1000 nm.

12. Composition selon l'une quelconque des revendications 9 à 11, caractérisée par le fait que le silicate mixte est un phyllosilicate.

13. Composition selon la revendication 12, caractérisee par le fait que le silicate mixte appartient à la famille des hectorites.

14. Composition selon la revendication 13, caractérisée par le fait que le silicate mixte est une laponite.

15. Composition cosmétique anti-rides selon l'une quelconque des revendications 9 à 14, caractérisée par le fait que le silicate mixte est présent à raison de 0,5 à 10 % en poids par rapport au poids total à la composition.

16. Composition anti-rides selon l'une quelconque des revendications 8 à 14, caractérisée par le fait qu'elle contient en outre un ou plusieurs principes actifs cosmétiques choisis parmi les agents anti-radicaux libres, les agents anti-UV, les agents hydratants, les vitamines, les protéines, les céramides, les $\alpha$-hydroxyacides, les $\beta$-hydroxyacides, les rétinoïdes et les polymères tenseurs organiques.

17. Composition anti-rides selon l'une quelconque des revendications 8 à 15, caractérisée par le fait qu'elle contient en outre au moins un adjuvant choisi parmi les solvants, les agents régulateurs de pH, les anti-oxydants, les agents séquestrants, les agents conservateurs, les pigments et colorants, les charges, les émollients, les agents anti-mousse, les huiles ou cires végétales ou animales, les silicones, les parfums, les agents tensioactifs, les plastifiants et les polymères épaississants ou gélifiants.

18. Composition anti-rides selon l'une quelconque des revendications 8 à 16, caractérisée par le fait qu'elle se présente sous forme d'une solution aqueuse ou hydroalcoolique, d'une émulsion huile-dans-eau ou eau-dans-huile ou encore d'une émulsion multiple, d'un gel aqueux, d'une pâte, d'un stick, d'une mousse, d'une dispersion d'huile dans une phase aqueuse en présence de nanoparticules polymèriques telles que les nanocapsules ou nanosphères, ou en présence de vésicules lipidiques de type ionique et/ou non ionique.

19. Procédé de traitement non thérapeutique d'une peau ridée consistant à appliquer sur la ride une composition anti-rides selon l'une quelconque des revendications 9 à 18 en une quantité suffisante pour estomper la ride par effet tenseur.